# FASCICULE DE BREVET EUROPEEN

(11) **EP 1 762 238 B1**
(45) Date de publication et mention de la délivrance du brevet: **10.08.2011**
(21) Numéro de dépôt: 06291423.9
(22) Date de dépôt: 08.09.2006
(51) Int. Cl.: A61K 31/165, A61P 25/24, A61K 31/22

(54) **Association entre l'agomélatine et un agent thymorégulateur et compositions pharmaceutiques les contenant**
Kombination von Agomelatin mit einem stimmungsstabilisierenden Wirkstoff und pharmazeutische Zusammensetzungen, die sie enthalten
Association of agomelatine with a mood stabilizer, and pharmaceutical compositions comprising them

(30) Priorité: 09.09.2005 FR 0509208
(43) Date de publication de la demande: 14.03.2007
(60) Demande divisionnaire: 10011609.4
(73) Titulaire: Les Laboratoires Servier, 92415 Courbevoie Cedex (FR)
(72) Inventeur: de Bodinat, Christian, 92210 Saint-Cloud (FR)

(56) Documents cités:
- JD GUELFI AND JR CALABRESE: "Efficacy and safety of the new antidepressant agomelatine in combination with a mood stabilizer in bipolar I patients with a major depressive episode" BIPOLAR DISORDERS, vol. 7, no. suppl.2, juin 2005 (2005-06), page 62, XP002378845
- CHILMAN-BLAIR K ET AL: "AGOMELATINE. ANTIDEPRESSANT TREATMENT OF BIPOLAR DISORDER MELATONIN AGONUST/5-HT20 ANTAGONIST" DRUGS OF THE FUTURE, BARCELONA, ES, vol. 28, no. 1, janvier 2003 (2003-01), pages 7-13, XP009036058 ISSN: 0377-8282

## Description

La présente invention concerne une nouvelle association entre l'agomélatine ou *N*-[2-(7-méthoxy-1-naphtyl)éthyl]acétamide de formule (I) : 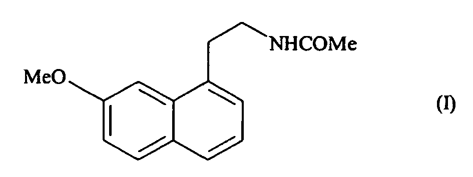 ou un de ses hydrates, formes cristallines et sels d'addition à un acide ou à une base pharmaceutiquement acceptable, et un agent thymorégulateur pour l'obtention de compositions pharmaceutiques utiles dans le traitement des troubles de l'humeur choisis parmi le trouble dépressif majeur, le trouble cyclothymique et le trouble dysthymique.

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement du trouble dépressif majeur, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Le document J.D. Guelfi and J.R. Calabrese, Bipolar disorders, 7(2), Juin 2005, p.62 divulgue l'utilisation de l'agomélatine en thérapie complémentaire d'un agent thymorégulateur chez des patients souffrants de trouble bipolaire I avec épisode dépressif majeur.

La demanderesse a présentement découvert que l'agomélatine ou *N*-[2-(7-méthoxy-1-naphtyl)éthyl]acétamide ou un de ses hydrates, formes cristallines et sels d'addition à un acide ou à une base pharmaceutiquement acceptable, utilisée en association avec un agent thymorégulateur, possédait des propriétés intéressantes permettant de l'utiliser dans le traitement des troubles de l'humeur choisis parmi le trouble dépressif majeur, le trouble cyclothymique et le trouble dysthymique.

Les désordres du système nerveux central tels que les troubles de l'humeur affectent une population nombreuse et de tous âges. Ils sont ainsi nommés à cause de l'implication d'affects persistants positifs ou négatifs d'intensité suffisamment sévère pour engendrer des comportements mal adaptés.
Le trouble dépressif majeur est un trouble aigu et sévère de l'humeur caractérisé par de la tristesse, du pessimisme, des idées suicidaires, un ralentissement idéomoteur et des plaintes somatiques diverses. Extrêmement handicapant, il peut mener à la cessation de toute activité fonctionnelle ou sociale. A la souffrance du sujet déprimé lui-même s'ajoute la souffrance de l'entourage familial.
Le trouble cyclothymique est caractérisé par la survenue répétitive de variations thymiques en positif (humeur expansive) et en négatif (humeur dépressive) de sévérité moindre que celles du trouble bipolaire, avec une altération fonctionnelle moindre ; cependant la difficulté diagnostique est réelle et certains éléments laissent penser que le trouble cyclothymique ferait le lit du trouble bipolaire. L'association d'un antidépresseur et d'un thymorégulateur permet de contrôler ces variations thymiques et d'empêcher leur passage à un trouble bipolaire caractérisé.
Enfin, le trouble dysthymique est un trouble chronique et intense de l'humeur qui se caractérise par de longues périodes d'humeur dysphorique et d'altération fonctionnelle. Tout comme pour le syndrome de l'épuisement professionnel, les autres symptômes de la dysthymie peuvent comprendre les sentiments suivants : manque d'adaptation, désespoir, irritabilité ou colère excessive, culpabilité, perte d'intérêt ou de plaisir généralisée, retrait social, fatigue chronique, déclin de l'activité ou de la productivité et mauvaise concentration. La dysthymie est un trouble mental insidieux. Contrairement aux symptômes fonctionnels invalidants associés à des maladies comme le trouble dépressif majeur, les sujets atteints de dysthymie souffrent généralement d'un dysfonctionnement social et professionnel modéré. Par exemple, malgré des perturbations typiques dans leur fonctionnement interpersonnel général, on voit souvent des sujets dysthymiques travailler assidûment dans leur profession et maintenir une façade de normalité.

Bien qu'il existe un grand nombre de molécules effectives dans ce domaine - on citera plus particulièrement les thymoanaleptiques et les régulateurs thymiques - aucune ne permet de satisfaire pleinement ces différents états pathologiques, et nombre d'entre elles présentent d'importants effets secondaires. Ainsi, l'élaboration de nouveaux traitements alternatifs est toujours d'actualité et reste une nécessité.
La demanderesse a présentement découvert que, de façon surprenante, l'agomélatine ou un de ses hydrates, formes cristallines et sels d'addition à un acide ou à une base pharmaceutiquement acceptable, utilisée en association avec un agent thymorégulateur, possédait des propriétés tout à fait adaptées au traitement des troubles de l'humeur et plus particulièrement du trouble dépressif majeur, du trouble cyclothymique et du trouble dysthymique. Les thymorégulateurs, généralement décrits pour leurs propriétés antimaniaques leur permettant d'agir sur l'expansivité thymique des états maniaques, sont couramment utilisés dans le traitement des troubles bipolaires.
La demanderesse a maintenant découvert que ces agents thymorégulateurs présentent la particularité de potentialiser les effets de l'agomélatine tant dans le domaine des troubles dépressifs de l'humeur que dans celui des troubles maniaques.
Cet effet, non prévisible, permet d'envisager l'utilisation de l'association selon l'invention dans le traitement des troubles de l'humeur et plus particulièrement du trouble dépressif majeur, du trouble cyclothymique et du trouble dysthymique.

Parmi les agents thymorégulateurs selon l'invention, on peut citer plus particulièrement à titre non limitatif le lithium, la carbamazépine, le valproate et la lamotrigine, et plus préférentiellement le valproate.

L'invention concerne donc l'utilisation de l'association entre l'agomélatine ou un de ses hydrates, formes cristallines et sels d'addition à un acide ou à une base pharmaceutiquement acceptable, et un composé thymorégulateur, pour l'obtention de compositions pharmaceutiques destinées au traitement des troubles de l'humeur choisis parmi le trouble dépressif majeur, le trouble cyclothymique et le trouble dysthymique.

L'invention concerne également les compositions pharmaceutiques contenant l'association entre l'agomélatine ou un de ses hydrates, formes cristallines et sels d'addition à un acide ou à une base pharmaceutiquement acceptable, et un composé thymorégulateur, en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc.

Outre l'agomélatine et le composé thymorégulateur, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc.

### A titre d'exemple et de manière non limitative, on peut citer :

◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 1 mg et 50 mg d'agomélatine par 24 heures et plus préférentiellement 25 mg par jour. La dose de l'agent thymorégulateur sera moindre que celle utilisée lorsqu'il est administré seul.

### Composition pharmaceutique:

**Formule de préparation pour 1000 comprimés dosés à 25 mg :**

| | |
|---|---|
| N-[2-(7-méthoxy-1-naphtyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude clinique :

L'étude clinique réalisée utilise la nomenclature internationale, essentiellement le DSM-IV, ainsi que des outils de mesure validés tels que l'échelle de dépression d'Hamilton, l'échelle de manie de Young, l'échelle Impression Clinique Globale, tous instruments recommandés par les Guidelines en vigueur. L'association agomélatine-valproate versus placébo-thymorégulateur, permet de conclure dans les meilleures conditions méthodologiques à la supériorité de l'association.

## Revendications

1. Utilisation d'une composition pharmaceutique comprenant de l'agomélatine ou un de ses hydrates, formes cristallines et sels d'addition à un acide ou à une base pharmaceutiquement acceptable, en association avec un agent thymorégulateur pour la fabrication d'un médicament pour le traitement du trouble dépressif majeur, du trouble cyclothymique ou du trouble dysthymique.

2. Utilisation d'une association contenant de l'agomélatine ou un de ses hydrates, formes cristallines et sels d'addition à un acide ou à une base pharmaceutiquement acceptable et un agent thymorégulateur pour l'obtention de compositions pharmaceutiques destinées au traitement du trouble dépressif majeur, du trouble cyclothymique ou du trouble dysthymique.

## Claims

1. Use of a pharmaceutical composition comprising agomelatine, or one of its hydrates, crystalline forms and addition salts with a pharmaceutically acceptable acid or base, in association with a thymoregulatory agent in the manufacture of a medicament for the treatment of major depressive disorder, cyclothymic disorder or dysthymic disorder.

2. Use of an association containing agomelatine, or one of its hydrates, crystalline forms and addition salts with a pharmaceutically acceptable acid or base, and a thymoregulatory agent in obtaining pharmaceutical compositions intended for the treatment of major depressive disorder, cyclothymic disorder or dysthymic disorder.

## Patentansprüche

1. Verwendung einer pharmazeutischen Zubereitung, enthaltend Agomelatin oder eines seiner Hydrate, Kristallformen und Additionssalze mit einer pharmazeutisch annehmbaren Säure oder Base in Kombination mit einem thymoregulierenden Mittel für die Herstellung eines Arzneimittels zur Behandlung der Major-Depression, der Zyklothymie oder der Dysthymie.

2. Verwendung einer Kombination enthaltend Agomelatin oder eines seiner Hydrate, Kristallformen und Additionssalze mit einer pharmazeutisch annehmbaren Säure oder Base und einem Thymoregulator für die Herstellung von pharmazeutischen Zubereitungen zur Behandlung der Major-Depression, der Zyklothymie oder der Dysthymie.
